# EUROPEAN PATENT SPECIFICATION

(11) **EP 2 731 519 B1**
(45) Date of publication and mention of the grant of the patent: **22.08.2018**
(21) Application number: 11768176.7
(22) Date of filing: 13.07.2011
(51) Int. Cl.: A61B 17/34, A61B 17/00, A61B 17/02

(54) **DEVICE FOR PROTECTION AND NON-TRAUMATIC SUPPORT OF A SURGICAL CAVITY**
VORRICHTUNG ZUM SCHUTZ UND ZUR VERLETZUNGSFREIEN UNTERSTÜTZUNG EINER OPERATIONSHÖHLE
DISPOSITIF DE PROTECTION ET DE SUPPORT NON TRAUMATIQUE D'UNE CAVITÉ CHIRURGICALE

(43) Date of publication of application: 21.05.2014
(73) Proprietor: Rustia, Alessandro, 00185 Rome (IT)
(72) Inventor: Rustia, Alessandro, 00185 Rome (IT)
(74) Representative: Zizzari, Massimo
(86) International application number: PCT/IT2011/000243
(87) International publication number: WO 2013/008258

(56) References cited:
- EP-A1- 1 878 390
- DE-A1-102009 029 904
- US-A- 5 159 921
- US-A- 5 342 385
- US-A1- 2002 183 594
- US-A1- 2008 081 951
- US-A1- 2008 294 187

## Description

The present invention concerns a medical device, particularly suitable for protection and non-traumatic support of a surgical cavity, inside the body of a patient that is submitted to a surgical operation, in order to achieve an inner protected space where surgical instruments can be inserted.

As known, the current techniques of *surgery* include the use of specific instruments, so called *retractors,* in order to achieve and keep a space inside the body of a patient. These *retractors* are of different types and, usually, are composed of some real metallic pincers, that are attached at their distal end to an anatomical tissue of the same patient, in example to the edge of a skin layer, and at the other end to an outer structure or to a stiff frame. More of these *retractors* can be used in *great operations,* in order to keep an open and stable cavity and permit the insertion and the handling of surgical instruments inside the body of a patient.

DE 10 2009 029904 A1 discloses an inflatable retractor for providing access to a surgical site. This retractor is spirally shaped.

All the above *retractors* imply the serious drawback of keeping the anatomical tissues of the same patient, at points of attachment, under hard stretching, all during the time of operation. This fact cannot avoid to generate traumas and/or local injuries that, although are quickly recovered during the stay in the hospital, can cause pain and/or possible after-surgery complications for the patient.

Therefore, the present invention arises from the need to find some alternative ways to achieve an inner surgical space inside the body of a patient, that is stable and permits the insertion and handling of surgical instruments and decreases, at the same time, the possibility of direct or indirect damages to the patient. In fact, besides the above said injuries at the attaching points of *retractors,* some incidental traumas are possible, in example due to a sudden and unexpected motion of instruments and/or anatomical parts of the patient, and other traumas due to an operation in emergency conditions with low visibility, where in example it happens to have inner hemorrhage and/or diffusion of inner biological liquids that are not drained promptly.

Therefore, the main objective of the present invention is to disclose a specific medical device, for protection and non-traumatic support of a surgical cavity, that can be easily inserted in the body of a patient, and that can be adapted smoothly so that it can express a gradual and homogeneous pressure towards the tissues of the same patient, all along the outline of the contact wall.

Another objective is that the same device can be adapted, and the stiffness of the walls can be increased, on command, by inserting air in proper airtight chambers, contained inside the same contact walls.

Another further objective is that the same device includes some specific accessories, like in example: some *spatulas* (with respective housings) and some hooks suitable for a stable positioning of the same device inside the patient; some suction/drainage means so that the surgical space is kept free of possible operative and/or biological liquids, that can be lost; and means of lighting, so that the surgical space is kept enlightened and it is possible a direct vision of the area of intervention.

Therefore, it is specific subject of the present invention a device for protection and non-traumatic support of a surgical cavity according to claim 1. The present invention will now be described for illustrative but not limitative purposes, according to its preferred embodiments, with particular reference to figures of the enclosed drawings, wherein:
figure 1 is a upper lateral perspective view of a head of a patient to be submitted to a surgical operation;
figure 2 is a upper lateral perspective view of the same head of a patient of figure 1, where it is shown the use of a device for protection and non-traumatic support of a surgical cavity, according to the present invention;
figure 3 is a front view of a human brain, where the right and left hemispheres are kept separated and at a specific distance by two pincers, according to the prior art;
figure 4 is a front view of the same human brain of figure 3, where the right and left hemispheres are kept separated and at a specific distance by a device for protection and non-traumatic support of a surgical cavity, according to the present invention;
figures 5, 6 and 7 are a sequence upper lateral perspective views, of a device for protection and non-traumatic support of a surgical cavity, according to the present invention, following three respective increasing levels of inner air pressure, and therefore of stiffness of the entire structure;
figure 8 is a front perspective view of a patient submitted to a brain surgical operation, where it is represented the point of view of a surgeon who uses a surgical microscope;
figure 9 is a lateral perspective view of the same situation of figure 8, where it is better represented the point of view of a surgeon who uses a surgical microscope, with respective optical cone indicated.

It is here underlined that only few of the many conceivable embodiments of the present invention are described, which are just some specific non-limiting examples, having the possibility to describe many other embodiments based on the disclosed technical solutions of the present invention.

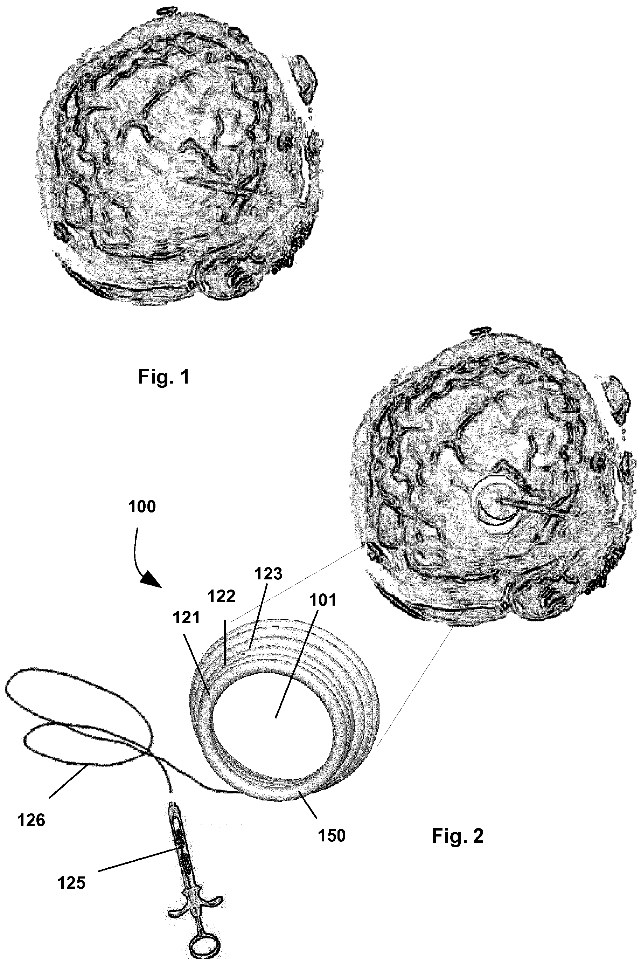

Figure 1 shows the head of a patient that should be submitted to a surgical operation, instead figure 2 shows the same patient with applied a device 100 for protection and non-traumatic support of a surgical cavity, according to the present invention. This device 100 includes a radial wall 150, composed of a set of elements 121, 122, 123, etc., aligned along the longitudinal axis and connected each other, or by a single element wrapping up the same longitudinal axis as a spiral or a double spiral, and having a previously defined shape, so that it can be adapted to a surgical working space created inside the body of a patient. The same radial wall 150 includes an inner air chamber, and means able to push air 125 from outside and keeping the same air inside. In such a way, the stiffness of the same radial wall 150 can be increased, and a previously defined pressure can be achieved, pushing tissues and inner anatomical parts of the same patient. That permits to achieve a protected surgical space 101 suitable to insert surgical instruments.

The above said inner air chamber can have a properly defined shape, in example having different volume profiles at respective *control points,* so that the progress of air insertion makes it possible to define, besides the stiffness of said wall 150, also the eccentricity of the entire structure, changing the shape of device 100 from a very elliptical shape to a nearly circular one, according to the operative needs of the moment.

The radial wall 150 can have a cylindrical shape, otherwise it can have a cone shape with an increasing diameter in the longitudinal direction, or it can have a cone shape with a decreasing diameter in the longitudinal direction.

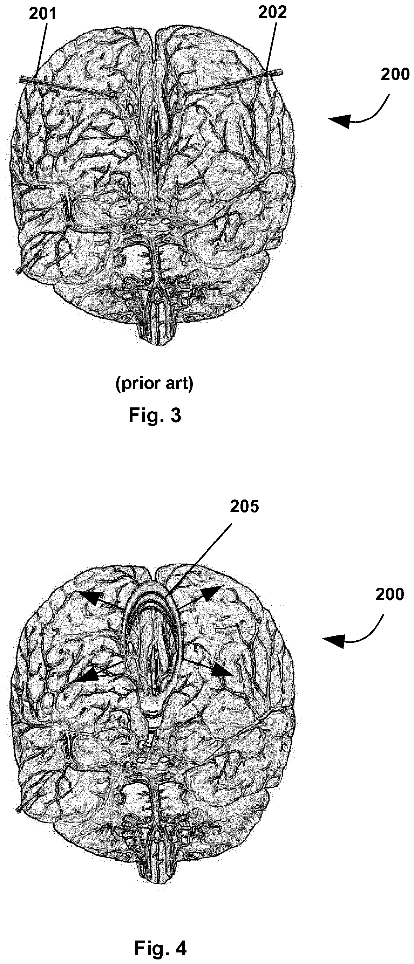

The example represented in figure 3 shows a situation where the hemispheres of brain 200 should be separated and kept at a specific distance, in order to operate inside the same brain. The procedure, according to prior art, involve some particular instruments, like in example elements 201 and 202, or some *retractors,* having their respective hook-shaped ends, that joint the edge of the brain tissue and keep it open all during the surgical operation.

Figure 4 shows how this function can be embedded by device of the present invention. In particular, a radial wall 205 expresses a specific pressure towards tissues and inner anatomical parts of the same patient, so that a protected surgical space 101 can be achieved in order to insert surgical instruments.

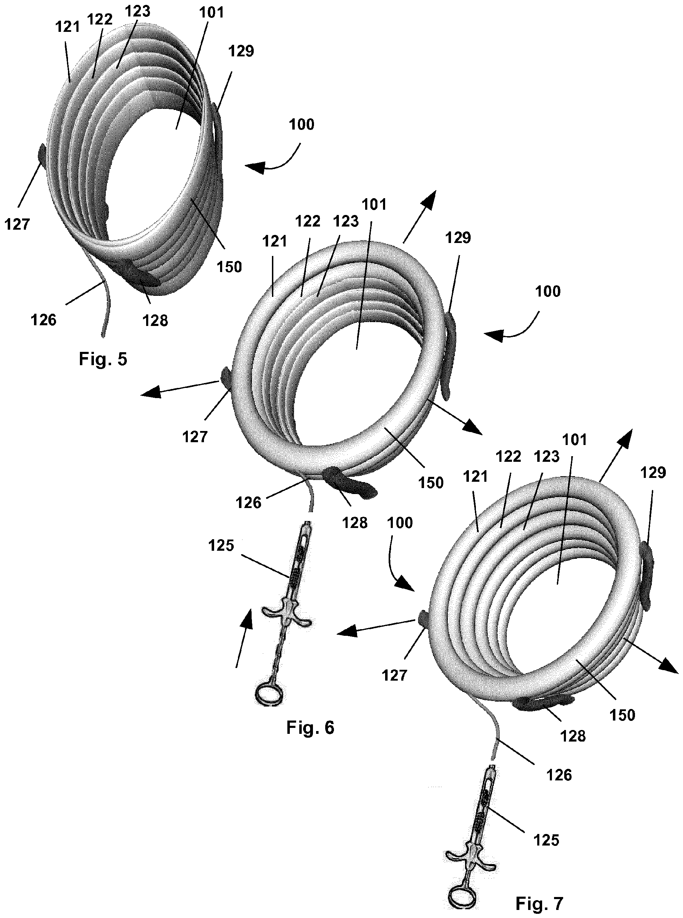

Figures 5, 6 and 7 represent a sequence of device 100 of invention, following three respective increasing levels of inner air pressure, and therefore of stiffness of the entire structure. Said means able to push air 125 are composed of a bolt syringe that, through a connecting tube 126 is jointed to the air chamber contained inside said radial wall 150. As an alternative, said means able to push air 125 are composed of more complex mechanical or electrical systems, able to detect a pressure level, inside the same radial wall 150, and to start/stop automatically the process of inflation/deflation of structure.

According to the invention, the radial wall 150 further includes means supporting the insertion of device 100 in the final position in the patient. The above means being composed of respective longitudinal channels 127, 128, 129, fixed along the profile of the radial wall 150, wherein some *spatulas* are housed. These *spatulas* having function of driving and supporting the insertion of device 100, and having the possibility to be removed later, when the same device 100 is stable in its final position.

Said radial wall 150 can further include means of anchorage in order to keep device 100 in its final position. Said means being composed of respective retractable hooks, fixed along the profile of said radial wall 150, that can be pulled inside, on command, afterwards when the same device 100 should be moved and/or removed because it is no more necessary for the operation.

The device 100 for protection and non-traumatic support of a surgical cavity 101, according to the present invention, can further include means for suction/drainage of liquids. These means can include in turn at least a tube of aspiration that reaches with its far end the open bottom of said radial wall 150. The above means being enabled/disabled on command, so that the surgical space is kept free of possible operative and/or biological liquids, that can be lost and can invade the area of intervention.

Furthermore, the device 100 can further include means of lighting, comprising in turn an optical fiber system that is wrapped as a spiral on said radial wall 150. This system is able to irradiate a widespread light, the above means being enabled/disabled on command, so that the surgical space is kept enlightened and it is possible a direct vision of the area of intervention.

Said radial wall 150 can be composed of an inert material, possibly transparent, not extendable or slightly extendable, so that a function of protection and non-traumatic support of a surgical cavity 101 can be embedded, and a direct vision of the area of intervention is possible along any direction.

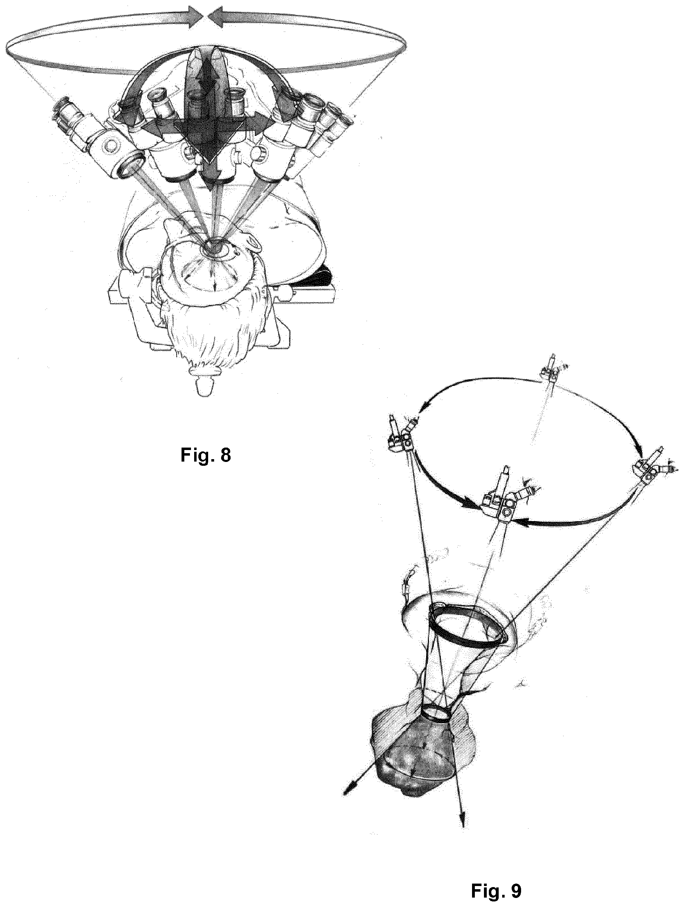

Figures 8 and 9 show, with reference to a patient submitted to a brain surgical operation, the point of view of a neuro-surgeon who uses a surgical microscope and watches from different angles, through a so called *key-hole,* in order to provide a proper visual exploration of the inner parts of the same patient, just by tipping the microscope forward, backward or on side. The present invention achieves the ideal *key-hole,* because it is non-traumatic and, according to its geometrical conformation and technical features, it protects the patient's tissues at 360 degrees, and in addition, changing the orientation of axis of the same *key-hole,* it is possible to change the axis of the surgical space of intervention, adapting it to any possible situation.

Therefore, the above examples show that the present invention achieves all the proposed objectives. In particular, it permits to achieve a specific medical device, for protection and non-traumatic support of a surgical cavity, that can be easily inserted in the body of a patient, and that can be adapted smoothly so that it can express a gradual and homogeneous pressure towards the tissues of the same patient, all along the outline of the contact wall.

In particular, the same device of invention can be adapted, and the stiffness of the walls can be increased, on command, by inserting air in proper airtight chambers, contained inside the same contact walls.

Another further characteristic of the present invention is that the same device includes some specific accessories, like in example: some spatulas (with respective housings) and some hooks suitable for a stable positioning of the same device inside the patient; some suction/drainage means so that the surgical space is kept free of possible operative and/or biological liquids, that can be lost; and means of lighting, so that the surgical space is kept enlightened and it is possible a direct vision of the area of intervention.

The present invention has been described for illustrative but not limitative purposes, according to its preferred embodiments, but it is clear that modifications and/or changes can be introduced by those skilled in the art without departing from the relevant scope, as defined in the enclosed claims.

## Claims

1. Device (100) for protection and non-traumatic support of a surgical cavity (101), comprising:
- a radial wall (150), composed of a set of elements (121, 122, 123, etc.), aligned along the longitudinal axis and connected each other, or by a single element wrapping up the same longitudinal axis as a spiral or a double spiral, and having a previously defined shape so that it can be adapted to a surgical working space created inside the body of a patient; the radial wall (150) including an inner air chamber, and means able to push air (125) from outside and keeping the same air inside, so that the stiffness of the radial wall (150) can be increased, and a previously defined pressure can be achieved, pushing tissues and inner anatomical parts of the same patient, and so that a protected surgical space (101) can be achieved in order to insert surgical instruments;
**characterized in that**
- said radial wall (150) further includes means supporting the insertion of device (100) in the final position in the patient, the above means being composed of respective longitudinal channels (127, 128, 129), fixed along the profile of the radial wall (150) on the outside surface of the radial wall (150), wherein some spatulas are inserted and housed, having function of driving and supporting the insertion of the entire device (100) when it is deflated; said channels (127, 128, 129), keeping a linear longitudinal profile later, in the inflated position, in order to enable said spatulas to be removed when the same device (100) is stable in the inflated position.

2. Device (100) for protection and non-traumatic support of a surgical cavity (101), **according to the previous claim, characterized in that:**
- said inner air chamber includes respective control points on surface so that when inflated it gives a specific shape and so that the insertion of air makes it possible to define, besides the stiffness of said wall (150), the eccentricity of the entire structure, changing device (100) from having a very elliptical shape to a nearly circular one, according to the operative needs of the moment.

3. Device (100) for protection and non-traumatic support of a surgical cavity (101), **according to one or more of previous claims, characterized in that:**
- said radial wall (150) has a cylindrical shape, or it has a cone shape with an increasing diameter in the longitudinal direction, or it has a cone shape with a decreasing diameter in the longitudinal direction.

4. Device (100) for protection and non-traumatic support of a surgical cavity (101), **according to one or more of previous claims, characterized in that:**
- said means able to push air (125) are composed of a bolt syringe that, through a connecting tube (126) is jointed to the air chamber contained inside said radial wall (150), or said means able to push air (125) are composed of more complex mechanical or electrical systems, able to detect a pressure level, inside the same radial wall (150) and to start/stop automatically the process of inflation/deflation of structure.

5. Device (100) for protection and non-traumatic support of a surgical cavity (101), **according to one or more of previous claims, characterized in that** it further comprises:
- means for suction/drainage of liquids, including at least a tube of aspiration that reaches with its far end the open bottom of said radial wall (150), the above means being enabled/disabled on command, so that the surgical space is kept free of possible operative and/or biological liquids, that can be lost and can invade the area of intervention.

6. Device (100) for protection and non-traumatic support of a surgical cavity (101), **according to one or more of previous claims, characterized in that** it further comprises:
- means of lighting, comprising an optical fiber system that is wrapped as a spiral on said radial wall (150), and that is able to irradiate a widespread light; the above means being enabled/disabled on command, so that the surgical space is kept enlightened and it is possible a direct vision of the area of intervention.

7. Device (100) for protection and non-traumatic support of a surgical cavity (101), **according to one or more of previous claims, characterized in that:**
- said radial wall (150) is composed of an inert material, that is transparent, not extendable or slightly extendable, so that a function of protection and non-traumatic support of a surgical cavity (101) can be embedded, and a direct vision of the area of intervention is possible along any direction.

## Patentansprüche

1. Vorrichtung (100) zum Schutz und zur atraumatischen Aufrechterhaltung eines chirurgischen Hohlraums (101), die folgendes umfasst:
- eine radiale Wand (150), die aus einer Reihe von Elementen (121, 122, 123, usw.) besteht, die nach der Längsachse ausgerichtet und miteinander verbunden sind, oder aus einem einzigen Element, das um dieselbe Längsachse gewickelt ist, wie eine Spirale oder eine Doppelspirale und eine vordefinierte Form hat, um sich perfekt einem chirurgischen Arbeitsplatz, der im Inneren des Körpers einen Patienten geschaffen wurde, anzupassen; die radiale Wand (150) umfasst in ihrem Inneren eine Luftkammer und Mittel zum Einführen von Luft (125) von außen und zur Beibehaltung derselben Luft im Inneren, um die Steifigkeiten der radialen Wand (150) zu erhöhen und daher einen vordefinierten Druck auf die Gewebe und die internen anatomischen Teile des Patienten auszuüben und so einen chirurgischen geschützten Raum (101) für die Einführung von chirurgischen Instrumenten zu erhalten,
der durch die Tatsache gekennzeichnet ist, dass:
- die zuvor genannte radiale Wand (150) weiter Mittel zum Unterstützen beim Einführen der Vorrichtung (100) in ihre Endposition im Inneren des Patienten umfasst, solche Mittel, die aus entsprechenden Längskanälen (127, 128, 129) bestehen, die entlang dem Profil der radialen Wand (150) im externen Bereich der radialen Wand (150) verbunden sind, in welche entsprechende Spachtel eingefügt sind und Platz finden, die als Führung und Stütze der gesamten Vorrichtung (100) funktionieren, wenn sie luftleer ist; wobei besagte Längskanäle (127, 128, 129) im Anschluss ein lineares Profil definieren, in der aufgepumpten Position, um denselben Spachteln zu erlauben, entfernt zu werden, wenn dieselbe Vorrichtung (100) stabil die aufgeblasene Position erreicht hat.

2. Vorrichtung (100) zum Schutz und zur atraumatischen Aufrechterhaltung eines chirurgischen Hohlraums (101), gemäß dem vorherigen Anspruch, ausgezeichnet durch die Tatsache, dass:
- die zuvor genannte Luftkammer jeweils Kontrollpunkte auf der Oberfläche umfasst, damit sie, wenn sie aufgeblasen ist, eine spezifische Form gibt und so das Einführen von Luft es möglich macht, neben der Steifigkeit besagter Wand (150), auch die Exzentrizität der gesamten Struktur zu definieren und die Vorrichtung (100) von einer sehr elliptischen Form zu einer fast kreisförmigen Form ändert, je nach Operationsanforderungen des Moments.

3. Vorrichtung (100) zum Schutz und zur atraumatischen Aufrechterhaltung eines chirurgischen Hohlraums (101), gemäß einem oder mehreren der vorherigen Ansprüche, ausgezeichnet durch die Tatsache, dass:
- die zuvor genannte radiale Wand (159) eine zylindrische Form oder eine konische Form mit zunehmendem Durchmesser in der Längsrichtung oder eine konische Form mit abnehmendem Durchmesser in der Längsrichtung hat.

4. Vorrichtung (100) zum Schutz und zur atraumatischen Aufrechterhaltung eines chirurgischen Hohlraums (101), gemäß einem oder mehreren der vorherigen Ansprüche, ausgezeichnet durch die Tatsache, dass:
- die zuvor genannten Mittel zum Einführen von Luft (125) aus einer Injektionsschraube bestehen, die über ein Verbindungsrohr (126) mit der Luftkammer im Inneren der radialen Wand (150) verbunden ist oder die Mittel zum Einführen von Luft (125) aus komplexeren mechanischen oder elektronischen Systemen bestehen, die in der Lage sind, das Druckniveau innerhalb der radialen Wand (150) zu erfassen und automatisch, mit Hilfe von Ventilen, den Inflations-/Deflationsprozess der Struktur zu starten und/oder zu blockieren.

5. Vorrichtung (100) zum Schutz und zur atraumatischen Aufrechterhaltung eines chirurgischen Hohlraums (101), gemäß einem oder mehreren der vorherigen Ansprüche, ausgezeichnet durch die Tatsache, zusätzlich folgendes zu umfassen:
- Mittel zum Ansaugen/Dränage von Flüssigkeiten, die wenigstens ein Ansaugrohr umfassen, das mit seinem Ende die geöffnete Basis der zuvor genannten radialen Wand (150) erreicht, diese Mittel können auf Befehl aktiviert/deaktiviert, um den chirurgischen Raum fei von möglichen Operations- und/oder biologischen Flüssigkeiten zu halten, die auftreten und in den Eingriffsbereich eindringen könnten.

6. Vorrichtung (100) zum Schutz und zur atraumatischen Aufrechterhaltung eines chirurgischen Hohlraums (101), gemäß einem oder mehreren der vorherigen Ansprüche, ausgezeichnet durch die Tatsache, zusätzlich folgendes zu umfassen:
- Mittel zur Beleuchtung, die ein System aus optischen Fasern umfassen, das spiralförmig entlang der radialen Wand (150) gewickelt ist und das in der Lage ist, diffuses Licht auszustrahlen; dieses System kann auf Befehl aktiviert/deaktiviert werden, um so den chirurgischen Raum gut zu beleuchten und die direkte Ansicht des gesamten Eingriffsbereichs zu begünstigen.

7. Vorrichtung (100) zum Schutz und zur atraumatischen Aufrechterhaltung eines chirurgischen Hohlraums (101), gemäß einem oder mehreren der vorherigen Ansprüche, ausgezeichnet durch die Tatsache, dass:
- die zuvor genannte radiale Wand (150) aus einem inerten Material besteht, das transparent, nicht dehnbar oder leicht dehnbar ist, um so eine atraumatische Schutz- und Aufrechterhaltungsfunktion eines chirurgischen Hohlraums (101) zu implementieren und die Beleuchtung des Eingriffsbereichs in jede Richtung zu begünstigen.

## Revendications

1. Dispositif (100) de protection et de maintien non traumatique d'une cavité chirurgicale (101), comprenant:
- une paroi radiale (150), constituée d'une série d'éléments (121, 122, 123, etc.), alignés selon l'axe longitudinal et reliés entre eux, ou d'un seul élément enroulé autour du même axe longitudinal comme une spirale ou une double spirale, et ayant une forme prédéfinie qui s'adapte parfaitement à un espace de travail chirurgical créé dans le corps d'un patient ; la paroi radiale (150) contient une chambre à air et des moyens d'injection d'air (125) de l'extérieur et de maintien de cet air à l'intérieur, afin d'augmenter la rigidité de la paroi radiale (150) et d'exercer une pression prédéfinie sur les tissus et les parties anatomiques internes du patient, et de manière à obtenir un espace chirurgical protégé (101) pour insérer des instruments chirurgicaux,
**caractérisé par le fait que**:
- ladite paroi radiale (150) comprend également des moyens de support pour l'insertion du dispositif (100) dans sa position finale à l'intérieur du patient, ces moyens étant constitués de canaux longitudinaux respectifs (127, 128, 129), reliés le long du profil de la paroi radiale (150) dans la partie extérieure de la paroi radiale (150), dans lesquels sont insérées et logées les spatules correspondantes, qui servent de guide et de support à l'ensemble du dispositif (100) lorsqu'il est dégonflé ; ces canaux longitudinaux (127, 128, 129) définissant ultérieurement un profil linéaire, en position gonflée, afin de permettre auxdites spatules d'être retirées lorsque le dispositif (100) a atteint sa position gonflée de façon permanente.

2. Dispositif (100) de protection et de maintien non traumatique d'une cavité chirurgicale (101), selon la revendication précédente, **caractérisé par le fait que**:
- ladite chambre à air comprend des points de contrôle respectifs sur la surface de telle sorte que lorsqu'elle est gonflée, elle donne une forme spécifique, et de telle sorte que l'injection d'air permet de définir, outre la rigidité de ladite paroi (150), l'excentricité de la structure entière, en modifiant le dispositif (100) d'une forme très elliptique à une forme presque circulaire, en fonction des besoins opérationnels du moment.

3. Dispositif (100) de protection et de maintien non traumatique d'une cavité chirurgicale (101), selon une ou plusieurs des revendications précédentes, **caractérisé par le fait que**:
- ladite paroi radiale (150) a une forme cylindrique, ou une forme conique avec un diamètre croissant dans le sens longitudinal, ou une forme conique avec un diamètres décroissant dans le sens longitudinal.

4. Dispositif (100) de protection et de maintien non traumatique d'une cavité chirurgicale (101), selon une ou plusieurs des revendications précédentes, **caractérisé par le fait que**:
- lesdits moyens d'injection d'air (125) sont composés d'une seringue à vis qui, à travers un tuyau de raccordement (126), est raccordée à la chambre à air contenue à l'intérieur de la paroi radiale (150), ou bien les moyens d'injection d'air (125) sont composés de systèmes mécaniques ou électroniques plus complexes, capables de détecter le niveau de pression à l'intérieur de la paroi radiale (150), et de démarrer et/ou arrêter automatiquement, au moyen de valves, le processus de gonflage/dégonflage de la structure.

5. Dispositif (100) de protection et de maintien non traumatique d'une cavité chirurgicale (101), selon une ou plusieurs des revendications précédentes, **caractérisé par le fait qu'**il comprend également:
- des moyens d'aspiration/drainage de liquides, comprenant au moins un tuyau d'aspiration, qui atteint avec son extrémité la base ouverte de ladite paroi radiale (150), ces moyens pouvant être activés/désactivés sur commande, de manière à maintenir l'espace chirurgical libre de tout liquide opérationnel et/ou biologique qui pourrait se présenter et envahir la zone d'intervention.

6. Dispositif (100) de protection et de maintien non traumatique d'une cavité chirurgicale (101), selon une ou plusieurs des revendications précédentes, **caractérisé par le fait qu'**il comprend également:
- des moyens d'éclairage, comprenant un système à fibres optiques enroulé en spirale le long de ladite paroi radiale (150) et en mesure d'émettre une lumière diffuse ; ce système pouvant être activé/désactivé sur commande, de manière à maintenir l'espace chirurgical bien éclairé et à voir directement toute la zone d'intervention.

7. Dispositif (100) de protection et de maintien non traumatique d'une cavité chirurgicale (101), selon une ou plusieurs des revendications précédentes, **caractérisé par le fait que**:
- ladite paroi radiale (150) est constituée d'un matériau inerte transparent, inextensible ou légèrement extensible, de manière à assurer une fonction de protection et de maintien non traumatique d'une cavité chirurgicale (101), et à faciliter l'éclairage de la zone d'intervention dans n'importe quelle direction.
